**Europäisches Patentamt**

**European Patent Office**

**Office européen des brevets**

(11) Veröffentlichungsnummer: **0 324 869 B1**

(12) **EUROPÄISCHE PATENTSCHRIFT**

(45) Veröffentlichungstag der Patentschrift: **10.06.92**

(21) Anmeldenummer: **88100652.2**

(22) Anmeldetag: **19.01.88**

(51) Int. Cl.⁵: **G01N  33/44**, G01N 11/10

(54) **Verfahren und Vorrichtung zum Prüfen des Aushärtungsgrades eines mit einem Reaktionsharz imprägnierten Trägers.**

(43) Veröffentlichungstag der Anmeldung:
**26.07.89 Patentblatt  89/30**

(45) Bekanntmachung des Hinweises auf die
Patenterteilung:
**10.06.92 Patentblatt  92/24**

(84) Benannte Vertragsstaaten:
**AT BE CH DE ES FR GB IT LI NL SE**

(56) Entgegenhaltungen:
**EP-A- 0 216 994**
**DE-A- 2 324 969**
**GB-A- 1 402 536**

**IBM TECHNICAL DISCLOSURE BULLETIN,**
**Band 26, Nr. 12, Mai 1984, New York, NY (US);**
**R.L.IMKEN, Seiten 6471-6472**

(73) Patentinhaber: **WERZALIT AG  +  CO.**
**Gronauer Strasse 70**
**W-7141 Oberstenfeld(DE)**

(72) Erfinder: **Golombek, Jörg**
**Eichhaldenstr. 73**
**W-7141 Oberstenfeld(DE)**

(74) Vertreter: **Bögl, Wolfgang, Dipl.-Ing.**
**Hölderlinstrasse 16**
**W-7121 Mundelsheim(DE)**

**Beschreibung**

Die Anmeldung betrifft ein Verfahren und eine Vorrichtung zum Prüfen des Aushärtungsgrades eines mit einem Reaktionsharz imprägnierten Trägers, bei dem das Reaktionharz bei einer bestimmten Temperatur aushärtet.

Mit einem Reaktionsharz imprägnierte Träger sind bekannt. Bei diesen kann es sich beispielsweise um Papierfolien oder Gewebebahnen handeln. Bei den bei einer bestimmten Temperatur aushärtenden Reaktionsharzen handelt es sich in der Regel um Duroplaste, beispielsweise Aminoplaste, wie Melaminharz, Phenolharz oder Harnstofformaldehydharz, oder Polyester und Epoxidharz.

Es ist üblich, den Zustand eines Reaktionsharzes mit A, B oder C zu kennzeichnen. Ein Reaktionharz im Zustand A ist flüssig ; es weist kurze Moleküle auf. Im Zustand B enthält das Reaktionsharz kein Lösungsmittel mehr; ein geringer Teil ist bereits kondensiert. Im Zustand C sind die Moleküle unendlich lang, und sie sind irreversibel miteinander vernetzt. (Siehe "Die Kunstoffe und ihre Eigenschaften"; Haus Dominghaus; VDI-Verlag, Düsseldorf; S.368-371)

Bei den mit einem Reaktionsharz imprägnierten Trägern befindet sich das Reaktionsharz im Zustand B. Solche imprägnierten Träger werden für verschiedene Zwecke weiterverarbeitet, beispielsweise werden aus einem nicht steigfähigen Gemisch von Fasern und einem wärmehärtenden Bindemittel vorgepreßte Profilkörper beim Heißpressen allseitig mit einer Schutz- und/oder Dekorschicht aus einem mit einem Reaktionsharz imprägnierten Träger umhüllt.

Da die Verarbeitbarkeit des mit dem Reaktionsharz imprägnierten Trägers von einer Mehrzahl von Parametern, wie dem Härteranteil im Reaktionsharz und den Eigenschaften des Trägers ( ph - Wert und Feuchtigkeitsgehalt ), abhängt, wäre es wünschenswert, ein Meßverfahren zu kennen, welches zuverlässige und reproduzierbare Meßergebnisse über die Eigenschaften von mit Reaktionsharz imprägnierten Trägern zu liefern vermag.

Das der Erfindung zugrunde liegende Problem besteht deshalb darin, ein solches Verfahren anzugeben.

Das Problem wird durch folgende Verfahrensschritte gelöst :

a. Eine Probe des imprägnierten Trägers wird in einem Halter eingespannt,

b. Die Probe wird in eine sich gegenüber dem imprägnierten Träger neutral verhaltende Flüssigkeit getaucht, welche eine Temperatur aufweist, bei welcher das Reaktionsharz aushärtet,

c. Die Probe wird in der Flüssigkeit bewegt und die für die Durchführung der Bewegung erforderliche Energie gemessen.

Aus den Meßwerten ergibt sich in Abhängigkeit von der Zeit ein Diagramm, welches für einen mit einem bestimmten Reaktionsharz imprägnierten Träger typisch ist und an dem bestimmte Eigenschaften des imprägnierten Trägers erkannt werden können.

Für die Durchführung des Verfahrens hat sich eine Vorrichtung als besonders geeignet erwiesen, welche gekennzeichnet ist durch einen, eine Prüfflüssigkeit aufnehmenden Behälter und einen oberhalb des Behälters heb- und senkbar angeordneten, mit einem für das Einspannen einer Probe des imprägnierten Trägers vorgesehenen und mit einem reversiblen Rotationsantrieb gekuppelten Meßkopf mit Probenhalter, bei dem der Probenhalter mit Probe in der abgesenkten Stellung in die Prüfflüssigkeit eintaucht.

Vorteilhafte Ausgestaltungen des erfindungsgemäßen Verfahrens sind in den Ansprüchen 2 bis 6 enthalten.

Die Erfindung ist nachstehend anhand der Figuren 1 bis 3 erläutert. Es zeigen :

Fig. 1    das beim Aushärten der Probe eines mit einem Reaktionsharz imprägnierten Trägers aufgenommene Diagramm,

Fig. 2    schematisch die Vorrichtung zur Aufnahme des Diagramms und

Fig. 3    den Verlauf des Motorstromes in Abhängigkeit von der Zeit, wenn der Halter mit der Probe des imprägnierten Trägers in der Flüssigkeit bewegt wird.

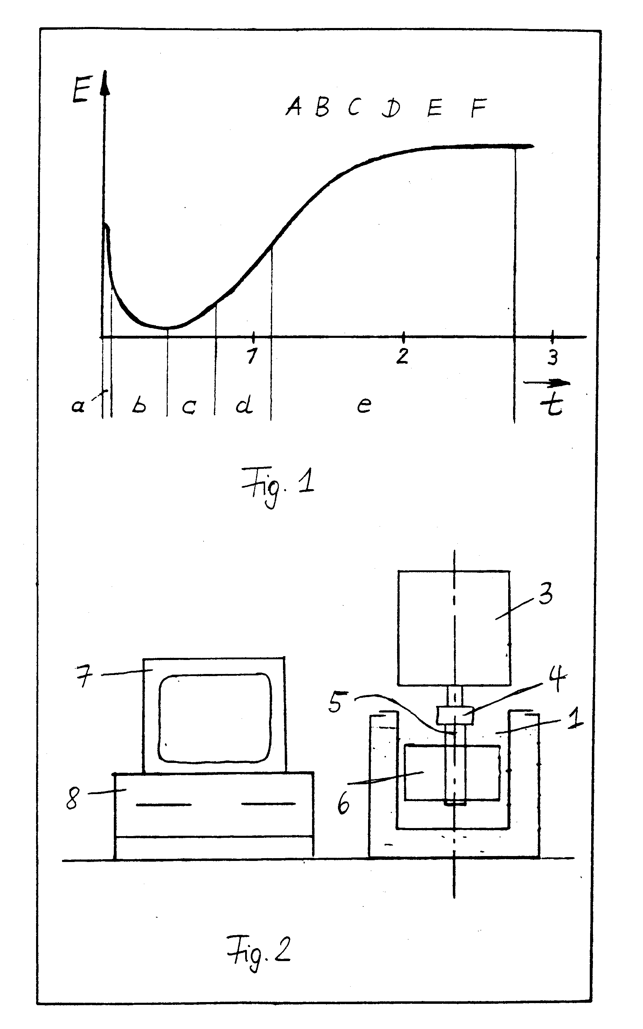

Der rechte Teil der aus Fig. 2 erkennbaren Vorrichtung enthält den für die Aufnahme der Prüfflüssigkeit vorgesehenen Behälter 1. Wenn mit dieser Vorrichtung die Daten eines mit einem Reaktionsharz imprägnierten Trägers ermittelt werden sollen, bei dem das Reaktionsharz bei sehr hohen Temperaturen, beispielsweise bei 200 ° C , aushärtet, dann ist der Behälter mit einer Heizung und einer Vorrichtung zur Konstanthaltung der Prüfflüssigkeitstemperatur ausgerüstet. Sollte es sich bei dem Reaktionsharz um ein solches handeln, welches bei niedrigen Temperaturen aushärtet, dann kann der Behälter auch mit einer Kühlung und einer Vorrichtung zur Konstanthaltung der niedrigen Prüfflüssigkeitstemperatur ausgerüstet sein.

Als Prüfflüssigkeit hat sich Silikonöl als geeignet erwiesen, welches chemisch nicht mit dem Reaktionsharz reagiert und bis auf 200 ° C erhitzt werden kann. Oberhalb des Behälters 1 ist heb- und senkbar der reversible Rotationsantrieb 3 angeordnet, dessen Abtriebswelle mit dem Meßkopf 4 gekuppelt ist. Der Rotationsantrieb soll in der Weise steuerbar sei, daß die Abtriebswelle bei eingeschaltetem Rotationsantrieb entweder nur in der gleichen Drehrichtung dreht oder ihre Drehrichtung von Zeit zu Zeit ändert. An dem Meßkopf 4 ist der Probenhalter 5 befestigt, an dem der blattförmige imprägnierte Träger 6 senkrecht eingespannt ist.

Mit dem im linken Teil der Fig. 2 dargestellten Bildschirm 7 und der elektronischen Rechenvorrichtung 8 soll deutlich gemacht werden, daß dort ein an einer, die gewünschten Eigenschaften aufweisenden Probe ermitteltes Diagramm, d.h. ein Idealdiagramm, gespeichert, erforderlichenfalls als Standbild wiedergegeben und mit einem an einer anderen Probe aufgenommenem Diagramm verglichen werden kann.

Die Fig. 1 zeigt den typischen Verlauf eines solchen Diagramms. Wie zu erkennen, weist die Probe, d.h. der mit einem Reaktionsharz impägnierte Träger, zum Zeitpunkt $t = 0$ eine bestimmte Steifigkeit auf. Die Steifigkeit der Probe nimmt zunächst sehr stark ab, bis sie zwischen b und c ihren geringsten , sehr niedrigen Wert erreicht hat. Zu diesem Zeitpunkt ist die Probe am weichsten. Danach nimmt die Steifigkeit kontinuierlich zu, bis sich das Diagramm asymptotisch einem maximalen Wert nähert.Das deutet darauf hin, daß von diesem Zeitpunkt an, welcher in Fig. 1 etwa bei $t = 3$ liegt, die Steifigkeit der Probe nicht mehr zunimmt, d.h. das Reaktionsharz ist ausgehärtet.

Aus dem Diagramm läßt sich eine Reihe von Parametern ablesen, welche für die Verarbeitung des imprägnierten Trägers wichtig sind. Der Bereich a des Diagramms hat keinen Einfluß auf die spätere Verarbeitbarkeit des impägnierten Trägers. Die Bereiche b und c des Diagramms sind repräntativ für das Fließverhalten des Reaktionsharzes. Anhand des Verlaufs des Diagramms in diesem Bereich kann auch erkannt werden, ob der imprägnierte Träger infolge zu langer Lagerung unbrauchbar geworden ist.

In dem Bereich d ist die Steilheit des Diagramms beispielsweise ein Maß für die Härterkonzentration im Reaktionsharz. Im Bereich e sind über dem Diagramm die verschiedenen Aushärtungsgrade A bis F eingetragen, wobei A den Zeitpunkt darstellt, an dem das Reaktionsharz im imprägnierten Träger nahezu vollständig ausgehärtet ist, welches für einige Anwendungsfälle erwünscht oder ausreichend ist.

Das in Fig. 1 gezeigt Diagramm ist in der Weise aufgenommen worden, daß der für das Bewegen der Probe in der Prüfflüssigkeit erforderliche elektrische Strom gemessen worden ist, und zwar vom Zeitpunkt des Eintauchens der Probe in die Prüfflüssigkeit bis zur vollständigen Aushärtung des Reaktionsharzes. Für die Aufnahme des in Fig. 1 gezeigten Diagrammes wurde die in die Prüfflüssigkeit eingetauchte Probe beispielsweise während einer Sekunde in die eine Drehrichtung gedreht. Danach wurde die Probe während drei Sekunden nicht bewegt und anschließend während einer Sekunde in die entgegengesetzte Drehrichtung gedreht.

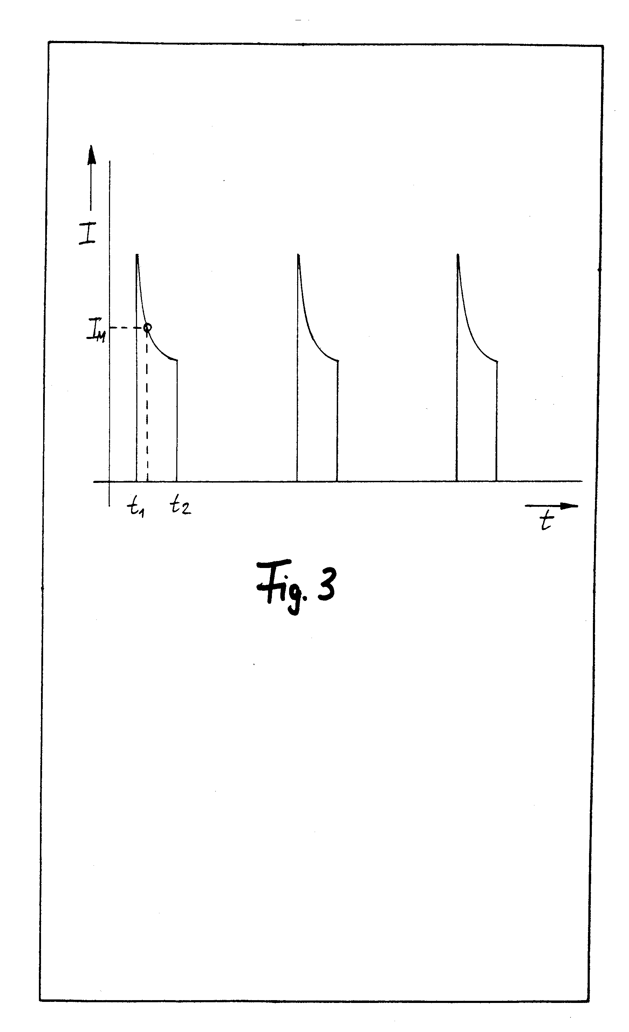

Der für das Bewegen der Probe erforderliche Strom I ist in Fig. 3 für drei aufeinanderfolgende Bewegungsvorgänge über der Zeit t aufgetragen. Wie aus Fig. 3 ersichtlich, wird zu Beginn einer jeden Bewegung, welche beispielsweise von $t_1$ bis $t_2$ dauert, ein relativ großer Strom benötigt. Das hat seine Ursache einerseits in dem Einschaltstrom des Motors und andererseits darin, daß die Massen der Probe und der diese umgebenden Prüfflüssigkeit erst beschleunigt werden müssen. Dieser Stromwert ist nicht repräsentativ für die Energie, welche für die Überwindung der Steifigkeit der Probe erforderlich ist. Der Strom nimmt gemäß Fig. 3 umsoweniger ab, je länger die Bewegung andauert. Der Stromwert zum Zeitpunkt $t_2$ ist sicherlich auch nicht repräsentativ für die Energie, welche für die Überwindung der Steifigkeit der Probe erforderlich ist. Je mehr sich die Bewegung dem Zeitpunkt $t_2$ nähert, umso stärker wirkt die kinetische Energie auf die Probe, welche durch die Beschleunigung der Probe und der diese umgebenden Prüfflüssigkeit entstanden ist.

Zur Herstellung des Diagramms, wie es aus Fig. 1 ersichtlich ist, wird deshalb der Stromwert $I_M$ gebildet, welcher dem Strom zu einem Zeitpunkt entspricht, wenn etwa ein Viertel bis ein Drittel der Einschaltdauer abgelaufen ist.

In der Praxis wird die Strommessung in der Weise durchgeführt, daß in der Zeitspanne zwischen $t_1$ und $t_2$ automatisch etwa dreihundert Messungen durchgeführt werden und der eine bestimmte Verzögerungs-Zeitspanne nach $t_1$ gemessene Stromwert für das Diagramm gemäß Fig. 1 genommen wird. Die Verzögerungs-Zeitspanne kann experimentell ermittelt werden; sie ist von den Eigenschaften der Vorrichtung abhängig, wie der Art des verwendeten Motors, der Viskosität der Prüfflüssigkeit und der Dimension der Probe.

Für die meisten praktischen Anwendungsfälle ergibt die Strommessung ausreichend genaue Werte für die zum Bewegen der Probe benötigte Energie. Sollten jedoch einmal genauere Werte benötigt werden, so ist auch eine Drehmomentmessung möglich. Diese ist mit an sich bekannten Mitteln durchführbar, erfordert aber einen größeren apparativen Aufwand.

Die Erfindung ist vorstehend anhand eines solchen Ausführungsbeispiels beschrieben worden, bei dem die Probe eine rotierende Bewegung ausführt. Es ist aber auch denkbar, daß die Probe in der Prüfflüssigkeit eine lineare Bewegung, beispielsweise eine Schwenk- oder Pendelbewegung ausführt.

Das erfindungsgemäße Verfahren und die Vorrichtung zur Durchführung des Verfahrens sind in mehrfacher Hinsicht bei der Verarbeitung von mit Reaktionsharz imprägnierten Trägern nützlich, beispielsweise von der Wareneingangsprüfung der angelieferten imprägnierten Träger bis zum Ermitteln der Verarbeitungsparameter.

**Patentansprüche**

1. Verfahren zum Prüfen des Aushärtungsgrades eines mit einem Reaktionsharz imprägnierten Trägers, bei dem das Reaktionsharz bei einer bestimmten Temperatur aushärtet, gekennzeichnet durch folgende Verfahrensschritte :
   a. Eine Probe des imprägnierten Trägers wird in einen Halter eingespannt,
   b. Die Probe wird in eine mit dem imprägnierten Träger nicht reagierende Flüssigkeit getaucht, welche eine Temperatur aufweist, bei welcher das Reaktionsharz aushärtet,
   c. Die Probe wird in der Flüssigkeit bewegt und die für die Durchführung der Bewegung erforderliche Energie gemessen.

2. Verfahren nach Anspruch 1, dadurch gekennzeichnet, daß das Bewegen der Probe in der Flüssigkeit in folgender Weise durchgeführt wird :
   a. Der Halter wird während einer bestimmten Zeitspanne in der einen Drehrichtung gedreht,
   b. Danach steht der Halter während einer bestimmten Zeitspanne still,
   c. Danach wird der Halter während einer bestimmten Zeitspanne in der zu a) entgegengesetzten Drehrichtung gedreht.

3. Verfahren nach den Ansprüchen 1 und 2, dadurch gekennzeichnet, daß die Stillstands - Zeitspanne des Halters ein Mehrfaches einer Bewegungszeitspanne beträgt.

4. Verfahren nach den Ansprüchen 1 bis 3, dadurch gekennzeichnet, daß der für das Bewegen der Probe benötigte elektrische Strom gemessen wird.

5. Verfahren nach den Ansprüchen 1 bis 4, dadurch gekennzeichnet, daß während der Bewegungszeitspanne eine Anzahl von Strommeßwerten ermittelt wird und aus diesen Meßwerten ein Meßwert gebildet wird, welcher etwa nach einem Viertel bis einem Drittel nach Beginn der Bewegungszeitspanne gemessen wurde.

6. Verfahren nach Anspruch 1 und wenigstens einem der Ansprüche 2 bis 5,
   dadurch gekennzeichnet, daß die Probe in der Flüssigkeit hin- und hergeschwenkt wird.

7. Vorrichtung zum Prüfen des Aushärtungsgrades eines mit einem Reaktionsharz imprägnierten Trägers,
   gekennzeichnet durch einen, eine Prüfflüssigkeit aufnehmenden Behälter ( 1 ) und einen oberhalb des Behälters ( 1 ) heb- und senkbar angeordneten, mit einem für das Einspannen einer Probe ( 6 ) des imprägnierten Trägers vorgesehenen und mit einem reversiblen Rotationsantrieb ( 3 ) gekuppelten Meßkopf ( 4 ) mit Probenhalter ( 5 ), bei dem der Probenhalter ( 5 ) mit Probe ( 6 ) in der abgesenkten Stellung in die Prüfflüssigkeit eintaucht.

**Claims**

1. Method of determining the solidification degree of a carrier impregnated with a reaction resin solidifiable at a predetermined temperature, **characterized** in that the method includes the following steps :
   a. A sample of the impregnated carrier is mounted on a holder;
   b. The sample is immersed into a fluid which does not react with the impregnated carrier, and has a temperature at which the reaction resin hardens;
   c. The sample is moved in the fluid, and the energy required for the movement of the sample is determined.

2. A method as claimed in claim 1, **characterized** in that the moving of the sample in said fluid is performed as follows :
   a. The holder is rotated during a predetermined period of time in the one direction of rotation;
   b. Then the holder is maintained immovable during a predetermined period of time;
   c. After that the holder is rotated during a predetermined period of time in a direction of rotation which is opposite to the direction of rotation as mentioned under a) above.

3. A method as claimed in claims 1 and 2, **characterized** in that the period of time during which said holder is maintained immovable is a multiple of the period of time of rotating said holder in any of said directions.

4. A method as claimed in claims 1 to 3, **characterized** in that the electric current which is required for the moving of said sample is measured.

5. A method as claimed in claims 1 to 4, **characterized** in that during said periods of time of moving said sample a number of current values is determined, and that from these values a measuring value is formed which was measured approximately after one-fourth to one-third after a beginning of the period of time of moving said sample.

6. A method as claimed in claim 1 and at least one of claims 2 to 5, **characterized** in that said sample is reciprocated in said fluid.

7. An arrangement for determining the solidification degree of a carrier impregnated with a reaction resin, **characterized** by a container ( 1 ) for receiving a test fluid, and by a measuring head ( 4 ) disposed above said container ( 1 ), and which is provided with a sample holder ( 5 ) capable of being lifted out and lowered into said container ( 1 ) and coupled with a reversible rotary drive ( 3 ), so that said holder ( 5 ) with a sample ( 6 ) of the impregnated carrier immerses into the test fluid in a lowered position of said measuring head ( 4 ) and holder ( 5 ).

**Revendications**

1. Procédé pour contrôler le degré de durcissement d'un substrat imprégné d'une résine de réaction, pour lequel la résine de réaction durcit à une température définie, caractérisé par les phases de procédé suivants :
    a. un échantillon du substrat imprégné est serré dans un support,
    b. l'échantillon est immergé dans un liquide qui ne réagit pas avec le substrat imprégné et qui présente une température à laquelle la résine de réaction durcit,
    c. l'échantillon est déplacé dans le liquide et l'énergie nécessaire à la réalisation de ce déplacement est mesurée.

2. Procédé selon la revendication 1, caractérisé en ce que le déplacement de l'échantillon dans le liquide est effectué de la façon suivante :
    a. le support est tourné pendant un intervalle de temps défini dans un sens,
    b. puis le support est arrêté pendant un intervalle de temps défini,
    c. le support est ensuite tourné pendant un intervalle de temps défini dans le sens inverse par rapport à a).

3. Procédé selon les revendications 1 et 2, caractérisé en ce que l'intervalle de temps d'arrêt du support est plusieurs fois supérieur à un intervalle de temps de déplacement.

4. Procédé selon les revendications 1 à 3, caractérisé en ce que le courant électrique nécessaire au déplacement de l'échantillon est mesuré.

5. Procédé selon les revendications 1 à 4, caractérisé en ce que, pendant l'intervalle de temps de déplacement, un certain nombre de valeurs de mesure de courant sont calculées, et qu'à partir de celles-ci est définie une valeur de mesure qui a été mesurée après l'écoulement d'environ un quart à un tiers de l'intervalle de temps de déplacement.

6. Proédé selon la revendication 1 et selon l'une au moins des revendications 2 à 5, caractérisé en ce que l'échantillon effectue un mouvement pivotant de va-et-vient dans le liquide.

7. Dispositif pour contrôler le degré de durcissement d'un substrat imprégné d'une résine de réaction, caractérisé par un récipient (1) contenant un liquide de contrôle, et par une tête de mesure (4) comportant un support d'échantillon (5), disposée, apte à être relevée et abaissée, au-dessus du récipient (1), prévue pour serrer un échantillon (6) du substrat imprégné et accouplée à un mécanisme d'entraînement en rotation réversible (3), ledit support d'échantillon (5) étant immergé, avec l'échantillon (6), en position abaissée, dans le liquide de contrôle.

Fig. 1

Fig. 2

Fig. 3